# EUROPEAN PATENT SPECIFICATION

(11) **EP 4 021 889 B1**
(45) Date of publication and mention of the grant of the patent: **26.06.2024**
(21) Application number: 20757595.2
(22) Date of filing: 20.08.2020
(51) Int. Cl.: C07C 315/06, C07C 317/14, B01D 9/00

(54) **A PROCESS FOR OBTAINING 4,4'-DICHLORODIPHENYL SULFONE**
VERFAHREN ZUR HERSTELLUNG VON 4,4-DICHLORDIPHENYLSULFON
PROCÉDÉ D'OBTENTION DE 4,4'-DICHLORODIPHÉNYL SULFONE

(30) Priority: 27.08.2019 EP 19193688; 06.02.2020 EP 20155783
(43) Date of publication of application: 06.07.2022
(73) Proprietor: BASF SE, 67056 Ludwigshafen am Rhein (DE)
(72) Inventor: HAMANN, Jessica Nadine, 67056 Ludwigshafen (DE); METZGER, Lukas, 67056 Ludwigshafen (DE); BLEI, Stefan, 67056 Ludwigshafen (DE)
(74) Representative: BASF IP Association
(86) International application number: PCT/EP2020/073371
(87) International publication number: WO 2021/037680

(56) References cited:
- WO-A1-2018/007481
- US-A- 2 067 043
- US-A- 3 634 043

## Description

The invention relates to a process for obtaining 4,4'-dichlorodiphenyl sulfone from an organic mixture comprising 4,4'-dichlorodiphenyl sulfone and a linear C₆ to C₁₀ carboxylic acid as organic solvent.

4,4-dichlorodiphenyl sulfone (in the following DCDPS) is used for example as a monomer for preparing polymers like polyether sulfone or polysulfone or as an intermediate of pharmaceuticals, dyes and pesticides.

DCDPS for example is produced by oxidation of 4,4'-dichlorodiphenyl sulfoxide which can be obtained by a Friedel-Crafts reaction of thionyl chloride and chlorobenzene as starting materials in the presence of a catalyst, for example aluminum chloride.

CN-A 108047101, CN-A 102351758, CN-B 104402780 and CN-A 104557626 disclose a two-stage process in which in a first stage a Friedel-Crafts acylation reaction is carried out to produce 4,4'-dichlorodiphenyl sulfoxide and in a second stage the 4,4'-dichlorodiphenyl sulfoxide is oxidized to obtain DCDPS in the presence of hydrogen peroxide. The oxidation reaction thereby is carried out in the presence of acetic acid. Such a process in which 4,4'-dichlorodiphenyl sulfoxide is produced in a first stage and DCDPS is obtained in a second stage using hydrogen peroxide in excess and acetic acid as solvent also is described in SU-A 765262.

Further processes for obtaining DCDPS by reacting chlorobenzene and thionyl chloride in a Friedel-Crafts reaction in a first stage to obtain 4,4'-dichlorodiphenyl sulfoxide and to oxidize the 4,4'-dichlorodiphenyl sulfoxide in a second stage using hydrogen peroxide as oxidizing agent and dichloromethane or dichloropropane as solvent are disclosed in CN-A 102351756 and CN-A 102351757.

A process for producing an organic sulfone by oxidation of the respective sulfoxide in the presence of at least one peroxide is disclosed in WO-A 2018/007481. The reaction thereby is carried out in a carboxylic acid as solvent, the carboxylic acid being liquid at 40°C and having a miscibility gap with water at 40°C and atmospheric pressure.

In all of these processes the DCDPS containing reaction product is cooled after the reaction is completed to precipitate solid DCDPS and to separate the solid DCDPS from the mixture.

Such a process for obtaining the solid DCDPS for example is a crystallization which is a well-known process to a skilled person and which is generally used for purifying organic substances. Crystallization processes for purifying organic compounds like acrylic acid or terephthalic acid for example are disclosed in DE-A 198 32 962 or US-A 2002/0198405 where for crystallization a solution comprising the organic substance is evaporated and the evaporated solvent is condensed. By this process the saturation point is shifted and crystallization starts. However, these processes only can be used for solutions based on a solvent which can be evaporated at process conditions at which the organic substance is not negatively affected.

Another process for crystallization to obtain an organic compound is disclosed in US-A 2009/0112040. Here, the cooling for crystallization is achieved by absorption and the cooled solvent is recycled into the crystallization apparatus.

It is an object of the present invention to provide a process for obtaining 4,4'-dichlorodiphenyl sulfone from an organic mixture comprising DCDPS and an organic solvent, which allows an efficient separation of the DCDPS from the organic solvent with a good yield, environmental sustainability and which is energy efficient.

This object is achieved by a process for obtaining DCDPS from an organic mixture comprising DCDPS and a linear C₆ to C₁₀ carboxylic acid as organic solvent, comprising:
(a) cooling the organic mixture by
   (a1a) mixing the organic mixture with water in a crystallization vessel to obtain a liquid mixture;
   (a1 b) cooling the liquid mixture obtained in (a1a) to a temperature below the saturation point of DCDPS by
      (i) reducing the pressure in the crystallization vessel to a pressure at which the water starts to evaporate,
      (ii) condensing the evaporated water by cooling
      (iii) mixing the condensed water into the liquid mixture in the crystallization vessel,to obtain a suspension comprising crystallized DCDPS;
      or by
   (a2) bringing the organic mixture into contact with at least one coolable surface and thereby reducing the temperature in the organic mixture with a cooling rate in the range from 5 to 50 K/h until a temperature in the range from 0 to 30°C is reached, wherein the organic mixture and the at least one coolable surface have a temperature difference which is kept during the whole cooling process in the range from 1 to 30 K to obtain a suspension comprising crystallized DCDPS;
(b) carrying out a solid-liquid-separation of the suspension obtained in (a1b) or (a2) to obtain a residual moisture containing solid DCDPS as product and mother liquor comprising the organic solvent and water.

The saturation point denotes the temperature of the liquid mixture at which DCDPS starts to crystallize. This temperature depends on the concentration of the DCDPS in the liquid mixture. The lower the concentration of DCDPS in the liquid mixture, the lower is the temperature at which crystallization starts.

The temperature of the organic mixture before starting cooling has to be such that the DCDPS comprised in the liquid mixture is completely dissolved. Therefore, the temperature of the organic mixture preferably is above the saturation point and if the organic mixture is obtained as a reaction product in an oxidization reaction of DCDPSO and an oxidizing agent, the temperature of the organic mixture before cooling corresponds to the temperature of the reaction mixture obtained in the oxidization reaction.

Cooling by adding water, reducing the pressure to evaporate water, condensing the water by cooling and recycle the condensed water and mix it into the liquid mixture allows for cooling the DCDPS comprising organic mixture without cooling surfaces onto which particularly at starting the cooling process crystallized DCDPS accumulates and forms a solid layer. This enhances the efficiency of the cooling process. Also, additional efforts to remove this solid layer can be avoided. Surprisingly, it further has been shown that the obtained DCDPS has a better color index. The color index is expressed as APHA number according to ASTM D1209: 2019 and for DCDPS produced by this process the APHA number is constantly below 40, even if the 4,4'-dichlorodiphenyl sulfoxide used for producing the DCDPS has APHA numbers above 100.

Particularly when organic solvents are used which have a boiling point above 150°C at 1 bar, for example carboxylic acids, cooling by reducing the pressure to evaporate organic solvent, to condense the evaporated organic solvent by cooling and recycling the condensed organic solvent back into the crystallization vessel would require a high energy consumption to achieve the necessary low pressures. Using higher temperatures to evaporate organic solvent for shifting the saturation point such that DCDPS crystallizes on the other hand would have a negative effect on the DCDPS; particularly a change in color of the DCDPS cannot be excluded. By mixing the organic mixture with water and to evaporate, condense and recycle the condensed water, it is possible to shift the saturation point by cooling without evaporating organic solvent at high temperatures or to reduce the pressure to very low values which is very energy consuming. Surprisingly, cooling and crystallization of DCDPS by adding water, reducing the pressure to evaporate water, condensing the water by cooling and recycle the condensed water and mix it into the liquid mixture even can be carried out when carboxylic acids are used as solvent which have a poor solubility in water. To allow pressure reduction to evaporate the water, the crystallization vessel for cooling and crystallization by mixing the organic mixture with water and to evaporate, condense and recycle the condensed water is a gastight closed vessel.

Surprisingly it further has been shown that the formation of a solid layer on coolable surfaces also can be reduced or even avoided, when in a cooling process by bringing the organic mixture into contact with at least one coolable surface the temperature in the organic mixture is reduced with a cooling rate in the range from 5 to 50 K/h until a temperature in the range from 0 to 30°C is reached, and the temperature difference between the organic mixture and the at least one coolable surface is kept in the range from 1 to 30 K during the whole cooling process.

To crystallize the DCDPS, it is preferred to provide crystal nuclei. To provide the crystal nuclei, it is possible to use dried crystals which are added to the liquid mixture or to add a suspension comprising particulate DCDPS as crystal nuclei. If dried crystals are used but the crystals are too big, it is possible to grind the crystals into smaller particles which can be used as crystal nuclei. Further, it is also possible to provide the necessary crystal nuclei by applying ultrasound to the liquid mixture.

If cooling is carried out by adding water, reducing the pressure to evaporate water, condensing the water by cooling and recycle the condensed water and mix it into the liquid mixture, the crystal nuclei preferably are generated in situ in an initializing step. The initializing step preferably comprises following steps before reducing pressure in step (i):
- reducing the pressure in the crystallization vessel such that the boiling point of the water in the liquid mixture is in the range from 80 to 95°C;
- evaporating water until an initial formation of solids takes place;
- increasing the pressure in the crystallization vessel and heating the liquid mixture in the crystallization vessel to a temperature in the range from 1 to 10°C below the saturation point of DCDPS.

By reducing the pressure in the crystallization vessel such that the water starts to evaporate at a temperature in the range from 80 to 95°C, more preferred in the range from 83 to 92°C, the following evaporation of water leads to a saturated solution and the precipitation of DCDPS. By the following pressure increase and heating the liquid mixture in the crystallization vessel to a temperature in the range from 1 to 10°C below the saturation point of DCDPS the solidified DCDPS starts to partially dissolve again. This has the effect that the number of crystal nuclei is reduced which allows producing a smaller amount of crystals with a bigger size. Further it is ensured that an initial amount of crystal nuclei remains in the crystallization vessel. Cooling, particularly by reducing the pressure, can be started immediately after a pre-set temperature within the above ranges is reached to avoid complete dissolving of the produced crystal nuclei. However, it is also possible to start cooling after a dwell time for example of 0.5 to 1.5 h at the pre-set temperature.

For generating the crystal nuclei in the initializing step, it is possible to only evaporate water until an initial formation of solids take place. It is also possible to entirely condense the evaporated water by cooling and to return all the condensed water into the crystallization vessel. The latter has the effect that the liquid mixture in the crystallization vessel is cooled and solid forms. A mixture of both approaches, where only a part of the evaporated and condensed water is returned into the crystallization vessel, is also viable.

Cooling (a1b) of the liquid mixture by reducing the pressure, evaporate water, condense the evaporated water by cooling and mixing the condensed water into the liquid mixture can be carried out batchwise, semi-continuously or continuously.

Particularly if cooling (a1b) is carried out in a batchwise process, the pressure reduction to evaporate water and thereby to cool the liquid mixture can be for example stepwise or continuously. If the pressure reduction is stepwise, it is preferred to hold the pressure in one step until a predefined rate in temperature decrease can be observed, particularly until the predefined rate is "0" which means that no further temperature decrease occurs. After this state is achieved, the pressure is reduced to the next pressure value. In this case the steps for reducing the pressure all can be the same or can be different. If the pressure is reduced in different steps, it is preferred to reduce the size of the steps with decreasing pressure. Preferably, the steps in which the pressure is decreased are in a range from 10 to 800 mbar, more preferred in a range from 30 to 500 mbar and particularly in a range from 30 to 300 mbar.

If the pressure reduction (ii) is continuously, the pressure reduction can be for example linearly, hyperbolic, parabolic or in any other shape, wherein it is preferred for a non-linear decrease in pressure to reduce the pressure in such a way that the pressure reduction decreases with decreasing pressure. If the pressure is reduced continuously, it is preferred to reduce the pressure with a rate from 130 to 250 mbar/h, particularly with a rate from 180 to 220 mbar/h. Moreover, the pressure can be reduced bulk temperature controlled by use of a process control system (PCS), whereby a stepwise linear cooling profile is realized.

Preferably, the pressure reduction (ii) is temperature controlled with a stepwise cooling profile from 5 to 25 K/h to approximate a constant supersaturation with increasing solid content and thus, more crystalline surface for growth.

If the cooling (a1b) and thereby the crystallization is carried out in a semi-continuous process, the pressure preferably is reduced stepwise, wherein the semi-continuous process for example can be realized by using at least one crystallization vessel for each pressure step, respectively temperature step. For cooling the liquid mixture, the liquid mixture is fed into a first crystallization vessel having the highest temperature and cooled to a first temperature. Then the liquid mixture is withdrawn from the first crystallization vessel and fed into a second crystallization vessel having a lower pressure. This process is repeated until the liquid mixture is fed into the crystallization vessel having the lowest pressure. As soon as the liquid mixture is withdrawn from one crystallization vessel, fresh liquid mixture can be fed into that crystallization vessel, wherein the pressure in the crystallization vessel preferably is kept constant. "Constant" in this context means that variations in pressure which depend on withdrawing and feeding liquid mixture into the respective crystallization vessel are kept as low as technically possible but cannot be excluded.

Besides carrying out the cooling (a1b) batchwise or semi-continuous, it is also possible to perform the cooling (a1b) continuously. If the cooling (a1b) and thus the crystallization of DCDPS is performed continuously, it is preferred to operate the cooling and crystallization stepwise in at least two steps, particularly in two to three steps, wherein for each step at least one crystallization vessel is used. If the cooling and crystallization is carried out in two steps, in a first step the liquid mixture preferably is cooled to a temperature in the range from 40 to 90°C and in a second step preferably to a temperature in the range from -10 to 50°C. If the cooling is operated in more than two steps, the first step preferably is operated at a temperature in the range from 40 to 90°C and the last step at a temperature in the range from -10 to 30°C. The additional steps are operated at temperatures between these ranges with decreasing temperature from step to step. If the cooling and crystallization is performed in three steps, the second step for example is operated at a temperature in the range from 10 to 50°C.

If the cooling (a1b) and crystallization is carried out continuously, a stream of the suspension is continuously withdrawn from the last crystallization vessel. The suspension then is fed into the solid-liquid-separation (b). To keep the liquid level in the crystallization vessels within predefined limits fresh liquid mixture comprising DCDPS, organic solvent and water can be fed into each crystallization vessel in an amount corresponding or essentially corresponding to the amount of suspension withdrawn from the respective crystallization vessel. The fresh liquid mixture either can be added continuously or batchwise each time a minimum liquid level in the crystallization vessel is reached.

Independently of being carried out batchwise or continuously, crystallization by reducing the pressure to evaporate water, condensing the water by cooling and recycle the condensed water and mix it into the liquid mixture preferably is continued until the solids content in the suspension in the last step of the crystallization is in the range from 5 to 50 wt%, more preferred in the range from 5 to 40 wt% and particularly in the range from 20 to 40 wt%, based on the mass of the suspension.

To achieve this solids content in the suspension, it is preferred to reduce the pressure in (i) until the suspension which is obtained by the cooling has cooled down to a temperature in the range from 10 to 30°C, preferably in the range from 15 to 30°C and particularly in the range from 20 to 30°C.

The pressure at which this temperature is achieved depends on the amount of water in the liquid mixture. Preferably, the amount of water mixed to the organic mixture in (a) is such that the amount of water in the liquid mixture is in the range from 10 to 60 wt% based on the total amount of the liquid mixture. More preferred, the amount of water mixed to the organic mixture in (a) is such that the amount of water in the liquid mixture is in the range from 10 to 50 wt% based on the total amount of the liquid mixture and, particularly, the amount of water mixed to the organic mixture in (a) is such that the amount of water in the liquid mixture is in the range from 15 to 35 wt% based on the total amount of the liquid mixture.

Even though the cooling (a1b) and crystallization can be carried out continuously or batchwise, it is preferred to carry out the cooling (a1b) and crystallization batchwise. Batchwise cooling and crystallization allows a higher flexibility in terms of operating window and crystallization conditions and is more robust against variations in process conditions.

To support cooling of the liquid mixture if cooling is carried out by reducing the pressure to evaporate water, condensing the water by cooling and recycle the condensed water and mix it into the liquid mixture it is further possible to provide the crystallization vessel with coolable surfaces for an additional cooling. The coolable surfaces for example can be a cooling jacket, cooling coils, half-pipe coils or cooled baffles like so called "power baffles". Surprisingly, forming of precipitations and fouling on coolable surfaces can be avoided or at least considerably reduced, if the additional cooling is started not before the temperature of the liquid mixture is reduced to a temperature in the range from 20 to 60°C, more preferred in a range from 20 to 50°C and particularly in a range from 20 to 40°C.

Cooling by bringing the organic mixture into contact with at least one coolable surface (a2) usually is carried out batchwise in a crystallization vessel until the desired end temperature is reached. If the organic mixture is cooled by bringing the organic mixture into contact with coolable surfaces, cooling is continued until a temperature in the range from 0 to 30°C is reached, preferably until a temperature in the range from 15 to 30°C is reached and particularly until a temperature in the range from 20 to 30°C is reached.

The coolable surface for cooling by bringing the organic mixture into contact with the at least one coolable surface also can be a cooling jacket, cooling coils, half-pipe coils or cooling baffles. To cool the at least one coolable surface, a cooling liquid flows through the cooling jacket, cooling coils, half-pipe coils or cooling baffles. The cooling liquid can be each cooling liquid known to a skilled person. Suitable cooling liquids for example are water, glycol, C10/13 alkylated benzene (for example available as Therminol^{®} ADX-10), hydrogenated terphenyl (for example available as Therminol^{®} 66), di-benzene-toluene (for example available as Marlotherm^{®} SH) or a silicon oil (for example available as Korasilon^{®} Oil M10).

The temperature of the cooling liquid is selected such that the temperature difference between the organic mixture and the at least one coolable surface is kept in the range from 1 to 30 K, more preferred in the range from 5 to 20 K and particularly in the range from 5 to 15 K. To keep the temperature difference between the organic mixture and the at least one coolable surface in this range, it is necessary to reduce the temperature of the cooling liquid during the cooling and crystallization (a2) corresponding to the cooling of the organic mixture.

Cooling thereby can be carried out by stepwise reduction of the temperature of the cooling liquid or by continuous reduction of the temperature of the cooling liquid. If the temperature of the cooling liquid is reduced stepwise, the temperature of the cooling liquid is reduced until a predefined temperature is reached and then kept on that temperature for a predefined time. After this time has elapsed, the temperature of the cooling liquid is reduced to the next predefined temperature and then kept on that temperature. This is repeated until the predefined temperature is reached. Independently of a stepwise or continuous reduction of temperature of the cooling liquid, the temperature of the cooling liquid is reduced such that the organic mixture is cooled with a cooling rate in the range from 5 to 50 K/h, more preferred in the range from 10 to 30 K/h and particularly in the range from 15 to 25 K/h. For a stepwise temperature reduction of the cooling liquid the cooling rate is determined by the difference of the temperature of the organic mixture at the start of the temperature reduction of the cooling liquid and the temperature of the organic mixture after the predefined time of keeping the cooling liquid at the predefined temperature has elapsed based on the time from starting the temperature reduction of the cooling liquid until the predefined time of keeping the cooling liquid at the predefined temperature has elapsed.

For cooling the cooling liquid, for example an external heat exchanger can be used. Further, before starting cooling (a2), it is necessary to heat the cooling liquid to achieve the temperature difference between the organic mixture and the at least one coolable surface. For heating preferably the same heat exchanger is used as for cooling. However, it is also possible to use one heat exchanger to heat the cooling liquid to the starting temperature and a second heat exchanger for cooling the cooling liquid during the cooling (a2).

Also for cooling (a2) it is preferred to mix the organic mixture with water before starting the cooling (a2). The amount of water preferably is such that the obtained mixture contains 10 to 60 wt% water before bringing the mixture into contact with the at least one coolable surface. More preferred, the amount of water is such that the obtained mixture contains 10 to 50 wt% water and particularly the amount of water is such that the obtained mixture contains 15 to 35 wt% water before bringing the mixture into contact with the at least one coolable surface.

The water which is mixed to the organic mixture preferably has a temperature in the range from 40 to 100°C, more preferred in the range from 60 to 100°C and particularly in the range from 80 to 100°C.

Like on cooling by reducing the pressure to evaporate water, condensing the water by cooling and recycle the condensed water and mix it into the liquid mixture, it has surprisingly shown that also by cooling using at least one coolable surface the obtained DCDPS has a better color index if the organic mixture is mixed with water.

After completing the cooling and crystallization, the process is finished and if the cooling and crystallization is carried out by reducing the pressure to evaporate water, condensing the water by cooling and recycle the condensed water and mix it into the liquid mixture the pressure preferably is set to ambient pressure, again. Following the cooling and crystallization, the suspension which formed by cooling the liquid mixture in the crystallization vessel is subjected to a solid-liquid separation. In the solid liquid separation process, the solid DCDPS formed by cooling is separated from the liquid phase.

Independently of whether the cooling and crystallization is carried out by reducing the pressure to evaporate water, condensing the water by cooling and recycle the condensed water and mix it into the liquid mixture or by bringing the organic phase into contact with at least one coolable surface, the suspension obtained by cooling and crystallization preferably is stirred in the crystallization vessel to avoid precipitation of the crystals formed by cooling (a). For stirring, it is possible to provide the crystallization vessel with a device for agitating, for example a stirrer, and to agitate the suspension in the crystallization vessel. Agitating preferably is operated such that the energy input by stirring is kept on a minimal level, which is high enough to suspend the crystals but prevents them from breakage. For this purpose, the mean energy input preferably is in the range from 0.2 to 0.8 W/kg, particularly in the range from 0.25 to 0.5 W/kg. Moreover, a stirring device is used which does not show high local energy dissipation input to prevent from attrition of the crystals. Suitable stirrers for example are axially conveying stirrers like oblique blade agitators or cross-arm stirrers or radially conveying agitators like flat blade agitators. The stirrer may have at least 2 blades, more preferred at least 4 blades. Particularly preferred is a stirrer having 4 to 8 blades, for example 6 blades.

Independently of whether the cooling and crystallization is performed continuously or batchwise, and independently of whether the cooling and crystallization is carried out by reducing the pressure to evaporate water, condensing the water by cooling and recycle the condensed water and mix it into the liquid mixture or by bringing the organic phase into contact with at least one coolable surface, the solid-liquid-separation (b) can be carried out either continuously or batchwise, preferably continuously.

If the cooling and crystallization (a) is carried out batchwise and the solid-liquid-separation is carried out continuously at least one buffer container is used into which the suspension withdrawn from the crystallization vessel is filled. For providing the suspension a continuous stream is withdrawn from the at least one buffer container and fed into a solid-liquid-separation apparatus. The volume of the at least one buffer container preferably is such that each buffer container is not totally emptied between two filling cycles in which the contents of the crystallization vessel is fed into the buffer container. If more than one buffer container is used, it is possible to fill one buffer container while the contents of another buffer container are withdrawn and fed into the solid-liquid-separation. In this case the at least two buffer containers are connected in parallel. The parallel connection of buffer containers further allows filling the suspension into a further buffer container after one buffer container is filled. An advantage of using at least two buffer containers is that the buffer containers may have a smaller volume than only one buffer container. This smaller volume allows a more efficient mixing of the suspension to avoid sedimentation of the crystallized DCDPS. To keep the suspension stable and to avoid sedimentation of solid DCDPS in the buffer container, it is possible to provide the buffer container with a device for agitating the suspension, for example a stirrer, and to agitate the suspension in the buffer container. Agitating preferably is operated such that the energy input by stirring is kept on a minimal level, which is high enough to suspend the crystals but prevents them from breakage. For this purpose, the mean energy input preferably is in the range from 0.2 to 0.8 W/kg, particularly in the range from 0.25 to 0.5 W/kg. Moreover, a stirring device is used which does not show high local energy dissipation input to prevent from attrition of the crystals.

If the cooling and crystallization (a) and the solid-liquid-separation (b) are carried out batchwise the contents of the crystallization vessel directly can be fed into a solid-liquid-separation apparatus as long as the solid-liquid separation apparatus is large enough to take up the whole contents of the crystallization vessel. In this case it is possible to omit the buffer container. It is also possible to omit the buffer container when cooling and crystallization and the solid-liquid-separation are carried out continuously. In this case also the suspension directly is fed into the solid-liquid-separation apparatus. If the solid-liquid separation apparatus is too small to take up the whole contents of the crystallization vessel, also for batchwise operation at least one additional buffer container is necessary to allow to empty the crystallization vessel and to start a new batch.

If the cooling and crystallization (a) is carried out continuously and the solid-liquid-separation (b) is carried out batchwise the suspension withdrawn from the crystallization vessel is fed into the buffer container and each batch for the solid-liquid-separation is withdrawn from the buffer container and fed into the solid-liquid-separation apparatus.

The solid-liquid-separation for example comprises a filtration, centrifugation or sedimentation. Preferably, the solid-liquid-separation is a filtration. In the solid-liquid-separation liquid mother liquor comprising organic solvent and water is removed from the solid DCDPS and residual moisture containing DCDPS (in the following also termed as "moist DCDPS") is obtained as product. If the solid-liquid-separation is a filtration, the moist DCDPS is called "filter cake".

To allow reuse of the water and/or the organic solvent, if the mother liquor obtained in the solid liquid separation (b) contains organic solvent and water, it is preferred to separate the mother liquor comprising the organic solvent and water into an aqueous phase and an organic phase comprising the organic solvent. If no water is added in the cooling and crystallization, the mother liquor may only comprise the organic phase. However, even if no water is mixed to the organic phase, the organic phase may contain water which was added during the oxidization reaction forming the DCDPS or formed as a by-product in the oxidization reaction. Therefore, also if no additional water was mixed into the organic phase, the mother liquor preferably is subjected to a phase separation to obtain an organic phase comprising the organic solvent and an aqueous phase.

To remove impurities from the aqueous phase and/or the organic phase, for example organic residues in the aqueous phase or residual water in the organic phase, it is possible to further purify the aqueous phase and/or the organic phase in following purification steps, for example one or more washing steps or distillation steps. Particularly preferably the organic solvent is recycled into a process for obtaining the organic mixture, particularly an oxidization reaction in which the DCDPS is produced.

The organic mixture comprising the DCDPS and organic solvent can be obtained by any process known to a skilled person. This organic mixture for example can be produced by mixing DCDPS and organic solvent, for example for purifying DCDPS by the inventive process. Preferably, the organic mixture is obtained by an oxidization reaction of 4,4'-dichlorodiphenyl sulfoxide and an oxidization agent which is carried out in the organic solvent. In this case the organic solvent of the organic mixture is the organic solvent which was used in the process for producing the DCDPS.

If the organic mixture is obtained by an oxidization reaction, it is particularly preferred that the DCDPS is produced by reacting a solution comprising 4,4'-dichlorodiphenyl sulfoxide and at least one C₆-C₁₀ carboxylic acid as organic solvent with an oxidizing agent to obtain a crude reaction product comprising 4,4'-dichlorodiphenyl sulfone, wherein the concentration of water in the reaction mixture is kept below 5 wt%.

By keeping the concentration of water below 5 wt% it is possible to use the linear C₆-C₁₀ carboxylic acid which is only slightly health hazardous and which has a good biodegradability.

Another advantage of using the linear C₆-C₁₀ carboxylic acid is that the linear C₆-C₁₀ carboxylic acid shows a good separability from water at low temperatures which allows separation of the linear C₆-C₁₀ carboxylic acid without damaging the product and which further allows recycling the linear C₆-C₁₀ carboxylic acid as solvent into the oxidation process.

In the process for producing DCDPS a solution comprising 4,4'-dichlorodiphenyl sulfoxide (in the following termed as DCDPSO) and at least one C₆-C₁₀ carboxylic acid (in the following termed as carboxylic acid) is provided. In this solution, the carboxylic acid serves as solvent. Preferably, the ratio of DCDPSO to carboxylic acid is in a range from 1 : 2 to 1 : 6, particularly in a range from 1 : 2.5 to 1 : 3.5. Such a ratio of DCDPSO to carboxylic acid is usually sufficient to completely solve the DCDPSO in the carboxylic acid at the reaction temperature and to achieve an almost full conversion of the DCDPSO forming DCDPS and further to use as little carboxylic acid as possible. The solution comprising DCDPSO and carboxylic acid preferably is heated to a temperature in the range from 70 to 110°C, more preferred to a temperature in the range from 80 to 100°C and particularly in the range from 85 to 95°C, for example 86, 87, 88, 89, 90, 91, 92, 93, 94°C, before adding the oxidizing agent.

To provide the solution, it is possible to feed DCDPSO and the carboxylic acid separately into a reactor and to mix the DCDPSO and the carboxylic acid in the reactor. Alternatively, it is also possible to mix the DCDPSO and the carboxylic acid in a separate mixing unit to obtain the solution and to feed the solution into the reactor. In a further alternative, DCDPSO and a part of the carboxylic acid are fed into the reactor as a mixture and the rest of the carboxylic acid is fed directly into the reactor and the solution is obtained by mixing the mixture of DCDPSO and part of the carboxylic acid and the rest of the carboxylic acid in the reactor.

The at least one carboxylic acid can be only one carboxylic acid or a mixture of at least two different carboxylic acids. Preferably the carboxylic acid is at least one aliphatic carboxylic acid. The at least one aliphatic carboxylic acid may be at least one linear or at least one branched aliphatic carboxylic acid or it may be a mixture of one or more linear and one or more branched aliphatic carboxylic acids. Preferably the aliphatic carboxylic acid is a C₆ to C₉ carboxylic acid, whereby it is particularly preferred that the at least one carboxylic acid is an aliphatic monocarboxylic acid. Thus, the at least one carboxylic acid may be hexanoic acid, heptanoic acid, octanoic acid, nonanoic acid or decanoic acid or a mixture of one or more of said acids. For instance, the at least one carboxylic acid may be n-hexanoic acid, 2-methyl-pentanoic acid, 3-methyl-pentanoic acid, 4-methyl-pentanoic acid, n-heptanoic acid, 2-methyl-hexanoic acid, 3-methyl-hexanoic acid, 4-methyl-hexanoic acid, 5-methyl-hexanoic acid, 2-ethyl-pentanoic acid, 3-ethyl-pentanoic acid, n-octanoic acid, 2-methyl-heptanoic acid, 3-methyl-heptanoic acid, 4-methyl-heptanoic acid, 5-methyl-heptanoic acid, 6-methyl-heptanoic acid, 2-ethyl-hexanoic acid, 4-ethyl-hexanoic acid, 2-propyl pentanoic acid, 2,5-dimethylhexanoic acid, 5,5-dimethylhexanoic acid, n-nonanoic acid, 2-ethyl-heptanoic acid, n-decanoic acid, 2-ethyl-octanoic acid, 3-ethyl-ocantoic acid, 4-ethyl-octanoic acid. The carboxylic acid may also be a mixture of different structural isomers of one of said acids. For instance, the at least one carboxylic acid may be isononanoic acid comprising a mixture of 3,3,5-trimethyl-hexanoic acid, 2,5,5-trimethyl-hexanoic acid and 7-methyl-octanoic acid or neodecanoic acid comprising a mixture of 7,7-dimethyloctanoic acid, 2,2,3,5-tetramethyl-hexanoic acid, 2,4-dimethyl-2-isopropylpentanoic acid and 2,5-dimethyl-2-ethylhexanoic acid. Particularly preferably, however, the carboxylic acid is a linear C₆-C₁₀ carboxylic acid and particularly n-hexanoic acid or n-heptanoic acid.

Heating of the solution comprising DCDPSO and the carboxylic acid can be carried out in the reactor in which the reaction for obtaining the crude reaction product takes place or in any other apparatus before being fed into the reactor. Particularly preferably, the solution comprising DCDPSO and the carboxylic acid is heated to the respective temperature before being fed into the reactor. Heating of the solution for example can be carried out in a heat exchanger through which the solution flows before being fed into the reactor or more preferred in a buffer container in which the solution is stored before being fed into the reactor. If such a buffer container is used, the buffer container also may serve as mixing unit for mixing the DCDPSO and the carboxylic acid to obtain the solution.

A heat exchanger for example can be used when the process is operated continuously. Heating of the solution in a buffer container can be carried out in a continuously operated process as well as in a batchwise operated process. If a heat exchanger is used for heating the solution, any suitable heat exchanger can be used, for example a shell and tube heat exchanger, a plate heat exchanger, a spiral tube heat exchanger, or any other heat exchanger known to a skilled person. The heat exchanger thereby can be operated in counter current flow, co-current flow or cross flow.

Besides heating by using a heating fluid which usually is used in a heat exchanger or for heating in a double jacket or heating coil, also electrical heating or induction heating can be used for heating the solution.

If the solution is heated in the buffer container, any suitable container which allows heating of the contents in the container can be used. Suitable containers for example are equipped with a double jacket or a heating coil. If the buffer container additionally is used for mixing the DCDPSO and the carboxylic acid, the buffer container further comprises a mixing unit, for example a stirrer.

For carrying out the reaction, the solution preferably is provided in a reactor. This reactor can be any reactor which allows mixing and reacting of the components fed into the reactor. A suitable reactor for example is a stirred tank reactor or a reactor with forced circulation, particularly a reactor with external circulation and a nozzle to feed the circulating liquid. If a stirred tank reactor is used, any stirrer can be used. Suitable stirrers for example are axially conveying stirrers like oblique blade agitators or cross-arm stirrers or radially conveying agitators like flat blade agitators. The stirrer may have at least 2 blades, more preferred at least 4 blades. Particularly preferred is a stirrer having 4 to 8 blades, for example 6 blades. For reasons of process stability and process reliability, it is preferred that the reactor is a stirred tank reactor with an axially conveying stirrer.

For controlling the temperature in the reactor, it is further preferred to use a reactor with heat exchange equipment, for example a double jacket or a heating coil. This allows additional heating or heat dissipation during the reaction and keep the temperature constant or in a predefined temperature range at which the reaction is carried out. Preferably, the reaction temperature is kept in a range from 70 to 110°C, more preferred from 80 to 100°C and particularly in a range from 85 to 95°C, for example 86, 87, 88, 8990, 91, 92, 93, 94°C.

To obtain DCDPS, the solution comprising DCDPSO and carboxylic acid is oxidized by an oxidizing agent. Therefore, the oxidizing agent preferably is added to the solution to obtain a reaction mixture. From the reaction mixture the crude reaction product comprising DCDPS can be obtained.

The oxidizing agent used for oxidizing DCDPSO for obtaining DCDPS preferably is at least one peroxide. The at least one peroxide may be at least one peracid, for example one or a mixture of two or more, such as three or more peracids. Preferably, the process disclosed herein is carried out in the presence of one or two, particularly in the presence of one peracid. The at least one peracid may be a C₁ to C₁₀ peracid, which may be unsubstituted or substituted, e.g. by linear or branched C₁ to C₅ alkyl or halogen, such as fluorine. Examples thereof are peracetic acid, performic acid, perpropionic acid, percaprionic acid, pervaleric acid or pertrifluoroacetic acid. Particularly preferably the at least one peracid is a C₆ to C₁₀ peracid, for example 2-ethyl-hexanoic peracid. If the at least one peracid is soluble in water, it is advantageous to add the at least one peracid as aqueous solution. Further, if the at least one peracid is not sufficiently soluble in water, it is advantageous that the at least one peracid is dissolved in the respective carboxylic acid. Most preferably, the at least one peracid is a linear C₆ to C₁₀ peracid which is generated in situ.

Particularly preferably, the peracid is generated in situ by using hydrogen peroxide (H₂O₂) as oxidizing agent. At least a part of the added H₂O₂ reacts with the carboxylic acid forming the peracid. The H₂O₂ preferably is added as an aqueous solution, for instance of 1 to 90 wt% solution, such as a 20, 30, 40, 50, 60 or 70 wt% solution, preferably as 30 to 85 wt% solution, particularly as a 50 to 85 wt% solution, each being based on the total amount of the aqueous solution. Using a highly concentrated aqueous solution of H₂O₂, particularly a solution of 50 to 85 wt%, for example of 70 wt%, based on the total amount of the aqueous solution, may lead to a reduction of reaction time. It may also facilitate recycling of the at least one carboxylic acid.

Particularly preferably, the at least one peracid is a linear C₆ or C₇ peracid which is generated in situ. To additionally reduce the reaction time and to add only a small amount of water to the reaction mixture, it is particularly preferred that the C₆-C₁₀ carboxylic acid is n-hexanoic acid or n-heptanoic acid and the hydrogen peroxide is a 50 to 85 wt% solution.

To avoid accumulation of the oxidizing agent and to achieve a constant oxidation of the DCDPSO, it is preferred to add the oxidizing agent continuously with a feed rate from 0.002 to 0.01 mol per mol DCDPSO and minute. More preferred, the oxidizing agent is added with a feed rate from 0.003 to 0.008 mol per mol DCDPSO and minute and particularly with a feed rate from 0.004 to 0.007 mol per mol DCDPSO and minute.

The oxidizing agent can be added with a constant feed rate or with a varying feed rate. If the oxidizing agent is added with a varying feed rate, it is for example possible to reduce the feed rate with proceeding reaction within the above described range. Further it is possible to add the oxidizing agent in several steps with a stop of adding oxidizing agent between the steps. In each step during adding the oxidizing agent, the oxidizing agent can be added with a constant feed rate or a varying feed rate. Besides a decreasing feed rate with proceeding reaction, it is also possible to increase the feed rate or to switch between increasing and decreasing feed rates. If the feed rate is increased or decreased, the change in feed rate can be continuously or stepwise. Particularly preferably, the oxidizing agent is added in at least two steps wherein the feed rate in each step is constant.

If the oxidizing agent is fed in at least two steps, it is preferred to add the oxidizing agent in two steps, wherein adding the oxidizing agent to the solution preferably comprises:
(A) adding 0.9 to 1.05 mol oxidizing agent per mol DCDPSO uniformly distributed to the solution at a temperature in the range from 70 to 110°C over a period from 1,5 to 5 h in a first step to obtain a reaction mixture;
(B) agitating the reaction mixture after completion of the first step at the temperature of the first step for 5 to 30 min without adding oxidizing agent;
(C) adding 0.05 to 0.2 mol oxidizing agent per mol DCDPSO to the reaction mixture at a temperature in the range from 80 to 110°C over a period of less than 40 min in a second step;
(D) agitating the reaction mixture after completion of the second step at the temperature of the second step for 10 to 30 min without adding oxidizing agent,
(E) heating the reaction mixture to a temperature in the range from 95 to 110°C and hold this temperature for 10 to 90 min to obtain a crude reaction product comprising DCDPS.

If the oxidation of DCDPSO is carried out in at least two steps, for converting the DCDPSO into DCDPS, the DCDPSO is oxidized by adding the oxidizing agent in the first and second steps to the solution comprising DCDPSO and carboxylic acid.

In the first step 0.9 to 1.05 mol oxidizing agent per mol DCDPSO are added uniformly distributed to the solution at a temperature in the range from 70 to 110°C over a period from 1,5 to 5 h. By adding the oxidizing agent over such a period an accumulation of the oxidizing agent can be avoided.

"Uniformly distributed" in this context means, that the oxidizing agent can be added either continuously at a constant feed rate or at periodically changing feed rates. Besides continuous periodically changing feed rates, periodically changing feed rates also comprise discontinuously changing periodical feed rates for example feed rates where oxidizing agent is added for a defined time, then no oxidizing agent is added for a defined time and this adding and not adding is repeated until the complete amount of oxidizing agent for the first step is added. The period in which the oxidizing agent is added, is in a range from 1,5 to 5 h, more preferred in a range from 2 to 4 h and particularly in a range from 2,5 to 3,5 h. By adding the oxidizing agent uniformly distributed over such a period, it can be avoided that oxidizing agent accumulates in the reaction mixture which may result in an explosive mixture. Additionally, by adding the oxidizing agent over such a period, the process can be scaled up in an easy way as this allows also in an upscaled process to dissipate the heat from the process. On the other hand, by such an amount decomposition of the hydrogen peroxide is avoided and thus the amount of hydrogen peroxide used in the process can be minimized.

The temperature at which the first step is carried out is in the range from 70 to 110°C, preferably in the range from 85 to 100 °C and particularly in the range from 90 to 95 °C. In this temperature range, a high reaction velocity can be achieved at high solubility of the DCDPSO in the carboxylic acid. This allows to minimize the amount of carboxylic acid and by this a controlled reaction can be achieved.

After the addition of the oxidizing agent in the first step is completed, the reaction mixture is agitated at the temperature of the first step for 5 to 30 min without adding oxidizing agent. By agitating the reaction mixture after completion of adding the oxidizing agent, oxidizing agent and DCDPSO which did not yet react are brought into contact to continue the reaction forming DCDPS for reducing the amount of DCDPSO remaining as impurity in the reaction mixture.

To further reduce the amount of DCDPSO in the reaction mixture, after completing of agitating without adding oxidizing agent, 0.05 to 0.2 mol oxidizing agent per DCDPSO, preferably 0.06 to 0.15 mol oxidizing agent per mol DCDPSO, and particularly 0.08 to 0.1 mol oxidizing agent per mol DCDPSO are added to the reaction mixture in the second step.

In the second step, the oxidizing agent preferably is added in a period from 1 to 40 min, more preferred in a period from 5 to 25 min and particularly in a period from 8 to 15 min. The addition of the oxidizing agent in the second step may take place in the same way as in the first step. Further, it is also possible to add the entire oxidizing agent of the second step at once.

The temperature of the second step is in the range from 80 to 110°C, more preferred in the range from 85 to100 °C and particularly in the range from 93 to 98°C. It further is preferred that the temperature in the second step is from 3 to 10°C higher than the temperature in the first step. More preferred the temperature in the second step is 4 to 8 °C higher than the temperature in the first step and particularly preferably, the temperature in the second step is 5 to 7°C higher than the temperature in the first step. By the higher temperature in the second step, it is possible to achieve a higher reaction velocity.

After addition of the oxidizing agent in the second step, the reaction mixture is agitated at the temperature of the second step for 10 to 20 min to continue the oxidation reaction of DCDPSO forming DCDPS.

To complete the oxidation reaction, after agitating at the temperature of the second step without adding oxidizing agent, the reaction mixture is heated to a temperature in the range from 95 to 110°C, more preferred in the range from 95 to 105°C and particularly in the range from 98 to 103°C and held at this temperature for 10 to 90 min, more preferred from 10 to 60 min and particularly from 10 to 30 min.

In the oxidizing process, particularly when using H₂O₂ as oxidizing agent, water is formed. Further, water may be added with the oxidizing agent. According to the invention, the concentration of the water in the reaction mixture is kept below 5 wt%, more preferred below 3 wt% and particularly below 2 wt%. By using aqueous hydrogen peroxide with a concentration of 70 to 85 wt% the concentration of water during the oxidization reaction is kept low. It even may be possible to keep the concentration of water in the reaction mixture during the oxidization reaction below 5 wt% without removing water by using aqueous hydrogen peroxide with a concentration of 70 to 85 wt%.

Additionally or alternatively, it may be necessary to remove water from the process for keeping the concentration of water in the reaction mixture below 5 wt%. To remove the water from the process, it is for example possible to strip water from the reaction mixture. Stripping thereby preferably is carried out by using an inert gas as stripping medium. If the concentration of water in the reaction mixture remains below 5 wt% when using aqueous hydrogen peroxide with a concentration of 70 to 85 wt% it is not necessary to additionally strip water. However, even in this case it is possible to strip water to further reduce the concentration.

Suitable inert gases which can be used for stripping the water are non-oxidizing gases and are preferably selected from the group consisting of nitrogen, carbon dioxide, noble gases like argon or any mixture of these gases. Particularly preferably, the inert gas is nitrogen.

Suitable inert gases which can be used for stripping the water are non-oxidizing gases and are preferably nitrogen, carbon dioxide, noble gases like argon or any mixture of these gases. Particularly preferably, the inert gas is nitrogen.

The amount of inert gas used for stripping the water preferably is in the range from 0 to 2 Nm³/h/kg, more preferably in the range from 0.2 to 1.5 Nm³/h/kg and particularly in the range from 0.3 to 1 Nm³/h/kg. The gas rate in Nm³/h/kg can be determined according to DIN 1343, January 1990 as relative gas flow. Stripping of water with the inert gas may take place during the whole process or during at least one part of the process. If water is stripped at more than one part of the process, between the parts stripping of water is interrupted. The interruption of stripping water is independent of the mode in which the oxidizing agent is added. For example, it is possible to add the oxidizing agent without any interruption and to strip the water with interruptions or to add the oxidizing agent in at least two steps and to strip the water continuously. Further it is also possible, to strip water only during the addition of oxidizing agent. Particularly preferably, the water is stripped by continuously bubbling an inert gas into the reaction mixture.

To avoid the formation of areas with different compositions in the reactor which may lead to different conversion rates of DCDPSO and thus to different yield and amounts of impurities, it is preferred to homogenize the reaction mixture during the first step and the second step. Homogenization of the reaction mixture can be performed by any method known to a skilled person, for example by agitating the reaction mixture. To agitate the reaction mixture, it is preferred to stir the reaction mixture. For stirring, any suitable stirrer can be used. Suitable stirrers for example are axially conveying stirrers like oblique blade agitators or cross-arm stirrers or radially conveying agitators like flat blade agitators. The stirrer may have at least 2 blades, more preferred at least 4 blades. Particularly preferred is a stirrer having 4 to 8 blades, for example 6 blades. For reasons of process stability and process reliability, it is preferred that the reactor is a stirred tank reactor with an axially conveying stirrer.

The temperature of the reaction mixture during the process can be set for example by providing a pipe inside the reactor through which a tempering medium can flow. Under the aspect of ease of reactor maintenance and/or uniformity of heating, preferably, the reactor comprises a double jacket through which the tempering medium can flow. Besides the pipe inside the reactor or the double jacket the tempering of the reactor can be performed in each manner known to a skilled person, for example by withdrawing a stream of the reaction mixture from the reactor, passing the stream through a heat exchanger in which the stream is tempered and recycle the tempered stream back into the reactor.

To support the oxidation reaction, it is further advantageous to additionally add at least one acidic catalyst to the reaction mixture. The acidic catalyst may be at least one, such as one or more, such as a mixture of two or three additional acids. An additional acid in this context is an acid which is not the carboxylic acid which serves as solvent. The additional acid may be an inorganic or organic acid, with the additional acid preferably being an at least one strong acid. Preferably, the strong acid has a pKₐ value from -9 to 3, for instance -7 to 3 in water. A person skilled in the art appreciates that such acid dissociation constant values, Kₐ, can be for instance found in a compilation such as in IUPAC, Compendium of Chemical Terminology, 2nd ed. "Gold Book", Version 2.3.3, 2014-02-24, page 23. The person skilled in the art appreciates that such pKₐ values relate to the negative logarithm value of the Kₐ value. it is more preferred that the at least one strong acid has a negative pKₐ value, such as from -9 to -1 or -7 to -1 in water.

Examples for inorganic acids being the at least one strong acid are nitric acid, hydrochloric acid, hydrobromic acid, perchloric acid, and/or sulfuric acid. Particularly preferably, one strong inorganic acid is used, in particular sulfuric acid. While it may be possible to use the at least one strong inorganic acid as aqueous solution, it is preferred that the at least one inorganic acid is used neat. Suitable strong organic acids for example are organic sulfonic acids, whereby it is possible that at least one aliphatic or at least one aromatic sulfonic acid or a mixture thereof is used. Examples for the at least one strong organic acid are para-toluene sulfonic acid, methane sulfonic acid or trifluormethane sulfonic acid. Particularly preferably the strong organic acid is methane sulfonic acid. Besides using either at least one inorganic strong acid or at least one organic strong acid, it is also possible to use a mixture of at least one inorganic strong acid and at least one organic strong acid as acidic catalyst. Such a mixture for example may comprise sulfuric acid and methane sulfonic acid.

The acidic catalyst preferably is added in catalytic amounts. Thus, the amount of acidic catalyst used may be in the range from 0.1 to 0.3 mol per mol DCDPSO, more preferred in the range from 0.15 to 0.25 mol per mol DCDPSO. However, it is preferred to employ the acidic catalyst in an amount of less than 0.1 mol per mol DCDPSO, such as in an amount from 0.001 to 0.08 mol per mol DCDPSO, for example from 0.001 to 0.03 mol per mol DCDPSO. Particularly preferably, the acidic catalyst is used in an amount from 0.005 to 0.01 mol per mol DCDPSO.

The oxidization reaction for obtaining DCDPS can be carried out as a batch process, as a semi continuous process or as a continuous process. Preferably, the oxidization reaction is carried out batchwise. The oxidation reaction can be carried out at atmospheric pressure or at a pressure which is below or above atmospheric pressure, for example in a range from 10 to 900 mbar(abs). Preferably, the oxidation reaction is carried out at a pressure in a range from 200 to 800 mbar(abs) and particularly in a range from 400 to 700 mbar(abs).

The oxidization reaction can be carried out under ambient atmosphere or inert atmosphere. If the oxidization reaction is carried out under inert atmosphere, it is preferred to purge the reactor with an inert gas before feeding the DCDPSO and the carboxylic acid. If the oxidization reaction is carried out under an inert atmosphere and the water formed during the oxidation reaction is stripped with an inert gas, it is further preferred that the inert gas used for providing the inert atmosphere and the inert gas which is used for stripping the water is the same. It is a further advantage of using an inert atmosphere that the partial pressure of the components in the oxidization reaction, particularly the partial pressure of water is reduced.

By the oxidization reaction, the organic mixture is obtained which comprises 4,4'-dichlorodiphenyl sulfoxide solved in a linear C₆ to C₁₀ carboxylic acid as organic solvent. As in the oxidization reaction a strong acid is used as catalyst, the organic mixture produced by a respective oxidization reaction also comprises the strong acid as impurity.

Each process step described above can be carried out in only one apparatus or in more than one apparatus depending on the apparatus size and the amount of compounds to be added. If more than one apparatus is used for a process step, the apparatus can be operated simultaneously or - particularly in a batchwise operated process - at different time. This allows for example to carry out a process step in one apparatus while at the same time another apparatus for the same process step is maintained, for example cleaned. Further, in that process steps where the contents of the apparatus remain for a certain time after all components are added, for example the oxidization reaction or the cooling steps, it is possible after feeding all compounds in one apparatus to feed the components into a further apparatus while the process in the first apparatus still continues. However, it is also possible to add the components into all apparatus simultaneously and to carry out the process steps in the apparatus also simultaneously.

An illustrative embodiment of the invention is shown in the figure and explained in more detail in the following description.

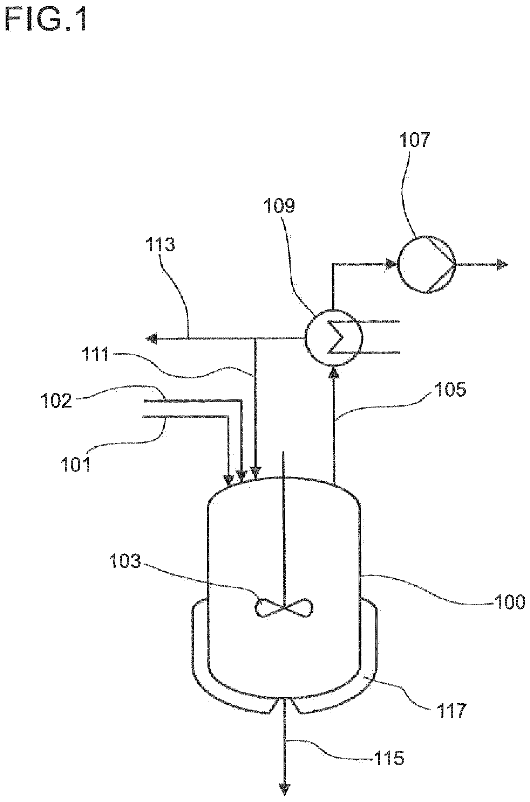

Figure 1 shows a crystallization vessel for crystallization of DCDPS by reducing the pressure to evaporate water, condensing the water by cooling and recycle the condensed water and mix it into the liquid mixture.

The only figure shows a crystallization vessel for crystallization of DCDPS by reducing the pressure to evaporate water, condensing the water by cooling and recycle the condensed water and mix it into the liquid mixture.

In one embodiment, crystallization of DCDPS is carried out in a crystallization vessel 100, which is a gastight closed vessel. The cooling and thereby the crystallization of DCDPS is performed by pressure reduction and lowering the boiling point of water in the liquid mixture due to the reduced pressure.

Via feed line 101 an organic mixture comprising DCDPS and an organic solvent is fed into the crystallization vessel 100. By a second feed line 102, additionally water is fed into the crystallization vessel 100. The crystallization vessel 100 preferably is a stirred tank comprising at least one stirrer 103. By stirring, the water is mixed into the organic mixture in the form of fine droplets, forming a liquid mixture. Further, by stirring the liquid mixture in the vessel, crystallized DCDPS is kept in the forming suspension and precipitation of crystallized DCDPS and thus fouling is avoided.

Besides adding the water via a second feed line 102 as shown in the figure, it is also possible to add the water into the feed line 101 and feed the organic mixture and the water together into the crystallization vessel 100. Further, the water and the organic mixture also can be fed into a mixing unit (not shown in figure 1) and from the mixing unit into the crystallization vessel 100.

For cooling the liquid mixture in the crystallization vessel 100 by dropping the boiling point of the water in the liquid mixture due to pressure reduction, an exhaust gas line 105 is provided which is connected to a vacuum pump 107. A suitable vacuum pump 107 for example is a liquid ring pump, vacuum steam jet pump or steam jet ejector. Between the crystallization vessel 100 and the vacuum pump 107, a condenser 109 is accommodated in the exhaust gas line 105. In the condenser 109 water which is evaporated from the boiling liquid mixture in the crystallization vessel 100 is condensed by cooling. The condensed water then is returned into the crystallization vessel 100 via line 111. Further, to remove low boilers from the crystallization a withdrawing line 113 is provided via which condensed water and low boilers if present, can be removed from the process.

By a drain line 115 a suspension comprising crystallized DCDPS is withdrawn from the crystallization vessel 100. The drain line 115 preferably is connected to a solid-liquid separation step, for example a filtration.

The crystallization vessel 100 for cooling and crystallization can be operated either batchwise or continuously. If the crystallization vessel 100 for cooling and crystallization is operated batchwise, in a first step the organic mixture and water are fed into the crystallization vessel 100, wherein the water and the organic mixture can be fed into the crystallization vessel 100 simultaneously or one after the other. After a predefined filling level is reached, feeding of the organic mixture and of the water is stopped. In a next step, the pressure in the crystallization vessel 100 is reduced using the vacuum pump 107 until a pressure in the crystallization vessel 100 is reached at which the boiling point of the water in the liquid mixture is in a range between 80 and 95°C. Due to pressure reduction the water in the liquid mixture starts boiling and water and low boilers evaporate. Once the saturation point of the DCDPS in the liquid mixture is reached, the pressure in the crystallization vessel 100 is increased and the liquid mixture is heated to a temperature between 85 and 100°C to dissolve partially the DCDPS to achieve crystal nuclei of a homogeneous size. After this heating phase, the pressure in the crystallization vessel 100 is reduced again. By this pressure reduction the boiling point of the water in the liquid mixture drops, water evaporates and is withdrawn from the crystallization vessel 100 via exhaust gas line 105. In the condenser 109 the evaporated water is condensed by cooling and the condensed water is recycled into the crystallization vessel 100. This recycling of water results in cooling of the liquid mixture leading to crystallization of DCDPSO. The temperature reduction in the crystallization vessel by pressure reduction and evaporation of the liquid is continued until the temperature in the vessel is in the range between 10 and 30°C, preferably ambient temperature.

After this temperature is reached, the pressure in the crystallization vessel 100 is increased until ambient pressure is reached without heating the liquid mixture. Therefore, the suspension produced in the crystallization vessel 100 preferably has ambient temperature and ambient pressure before it is withdrawn from the crystallization vessel 100 via drain line 115.

By this process for cooling the liquid in the crystallization vessel 100 no cooled surfaces have to be provided on which DCDPS would crystallize. Therefore, during crystallization no solid deposits on walls are formed. However, it is possible to support cooling of the liquid mixture by additional cooling after the temperature of the liquid mixture is reduced to a temperature in the range from 20 to 60°C. For this additional cooling for example coolable surfaces can be provided. These coolable surfaces for example can be a cooling jacket 117 as shown in the figure or alternatively or additionally cooling coils or cooling baffles.

If the crystallization vessel 100 is operated continuously, organic mixture and water are continuously fed into the crystallization vessel 100 via feed lines 101 and 102 and suspension comprising crystallized DCDPS and organic solvent is continuously removed from the crystallization vessel 100 via drain line 115. In a continuous process preferably at least two crystallization vessels 100 connected into series are used. In the first crystallization vessel 100 the pressure in the crystallization vessel is kept constantly at a value at which the temperature is in a range from 40 to 90°C and in the last crystallization vessel the pressure is kept such that the temperature is in the range from -10 to 50°C. If more than two crystallization vessels are used, the pressure in the crystallization vessels between the first and the last crystallization vessel is between the temperature in the first and in the last crystallization vessel and the temperature in all crystallization vessels decreases from the first to the last crystallization vessel. In each crystallization vessel 100 the temperature is set by withdrawing evaporated solvent via the exhaust gas line 105, condensing the evaporated solvent in the condenser 109 by cooling and returning the condensed solvent into the crystallization vessel 100 via line 111.

To keep a constant gas flow into the condenser 109 for continuous operation it is preferred to place an additional pump into the exhaust gas line 105 between the vessel 100 and the condenser 109 or into the line 111 between the condenser 109 and the vessel 100.

### Examples

### Example 1

For producing 4,4'-dichlorodiphenyl sulfone (DCDPS), 1098 g 4,4'-dichlorodiphenyl sulfoxide (DCDPSO) were dissolved in 2917 g n-heptanoic acid. Additionally, 7.2 g sulfuric acid were added. Over a period of 3h 197 g H₂O₂ were added to oxidize the DCDPSO in the solution.

To the resulting organic mixture comprising DCDPS and n-heptanoic acid, 881 g water were added with a temperature of 97°C. The thus obtained mixture was cooled by reducing the pressure according to the cooling profile shown in table 1.

**Table 1: cooling profile**

| time [h] | temperature [°C] | pressure [mbar] |
|---|---|---|
| 0:00 | 97 | 760 |
| 0:50 | 81 | 380 |
| 01:15 | 90 | 580 |
| 1:45 | 90 | 580 |
| 2:45 | 81 | 370 |
| 3:40 | 61,5 | 175 |
| 4:35 | 43 | 70 |
| 6:00 | 18 | 980 |

A suspension comprising 2480 g n-heptanoic acid and DCDPS was obtained by this process.

The suspension then was filtered at ambient temperature to obtain a filter cake comprising about 80 wt% DCDPS, 16 wt% n-heptanoic acid and 4 wt% water. The mother liquor which was separated off the filter cake in the filtration process contained about 78 wt% n-heptanoic acid, 20 wt% water and about 2,5 wt% DCDPS. For filtering the suspension, a glass nutsche was used which was covered with a Sefar^{®} Tetex DLW 17-80000-SK 020 Pharma filter cloth. For filtering, an absolute pressure of 500 mbar was set below the nutsche. After filtration, the filter cake was treated with dry air for 30 s.

The crystallizate obtained contained 99.724 wt% 4,4'-dichlorodiphenyl sulfoxide, 0.031 wt% 2,4'-dichlorodiphenyl sulfoxide and 0.24 wt% heptanoic acid and had a color index APHA=17.

### Example 2

For producing DCDPS, 1000.2 g DCDPSO were dissolved in 3000 g n-heptanoic acid. Additionally, 1.2 g sulfuric acid were added. Over a period of 3h 40 min 197.1 g H₂O₂ were added to oxidize the DCDPSO in the solution. The resulting organic mixture comprising DCDPS had a temperature of 93°C.

For separating the DCDPS, the organic mixture comprising DCDPS was cooled to a temperature of 20°C by a cooling ramp of 73 K in 2 h 38 min and then the temperature was maintained at 20 °C for additional 2h 34 min.

The suspension obtained by this process was filtered at ambient temperature to obtain a filter cake comprising the DCDPS. The filter cake was washed with 1.3 kg 5% aqueous NaOH and then two times with 1.3 kg water, each. The washed filter cake was dried for 16 h at a temperature of 60°C to obtain dried DCDPS.

The dried DCDPS had a color index APHA = 99 and a residual content of n-heptanoic acid of 0.23 wt%.

### Example 3

For producing DCDPS, 1000.3 g DCDPSO were dissolved in 3000 g n-heptanoic acid. Additionally, 1.3 g sulfuric acid were added. Over a period of 3h 40 min 197.1 g H₂O₂ were added to oxidize the DCDPSO in the solution. The resulting organic mixture comprising DCDPS had a temperature of 94°C.

To the resulting organic mixture comprising DCDPS and n-heptanoic acid, 794 g water were added with a temperature of 90°C. The thus obtained mixture was cooled by reducing the pressure over a time period of 5 h12 min in such a way that the temperature was reduced by 74 K. During cooling, the organic mixture was agitated.

The suspension obtained by this process was filtered at ambient temperature to obtain a filter cake comprising the DCDPS. The filter cake was washed with 1.3 kg 5% aqueous NaOH and then two times with 1.3 kg water, each. The washed filter cake was dried for 16 h at a temperature of 60°C to obtain dried DCDPS.

The dried DCDPS had a color index APHA = 31 and a residual content of n-heptanoic acid of 0.16 wt%.

## Claims

1. A process for obtaining 4,4'-dichlorodiphenyl sulfone from an organic mixture comprising 4,4'-dichlorodiphenyl sulfone and a linear C₆ to C₁₀ carboxylic acid as organic solvent, comprising:
(a) cooling the organic mixture by
(a1a) mixing the organic mixture with water in a crystallization vessel to obtain a liquid mixture;
(a1b) cooling the liquid mixture obtained in (a1a) to a temperature below the saturation point of 4,4'-dichlorodiphenyl sulfone by
(i) reducing the pressure in the crystallization vessel to a pressure at which the water starts to evaporate,
(ii) condensing the evaporated water by cooling,
(iii) mixing the condensed water into the liquid mixture in the crystallization vessel,
to obtain a suspension comprising crystallized 4,4'-dichlorodiphenyl sulfone;
or by
(a2) bringing the organic mixture into contact with at least one coolable surface and thereby reducing the temperature in the organic mixture with a cooling rate in the range from 5 to 50 K/h until a temperature in the range from 0 to 30°C is reached, wherein the organic mixture and the at least one coolable surface have a temperature difference which is kept during the whole cooling process in the range from1 to 30 K to obtain a suspension comprising crystallized 4,4'-dichlorodiphenyl sulfone.
(b) carrying out a solid-liquid-separation of the suspension obtained in (a1b) or in (a2) to obtain a residual moisture containing solid 4,4'-dichlorodiphenyl sulfone as product and mother liquor comprising the organic solvent and water.

2. The process according to claim 1, wherein the amount of water mixed to the organic mixture in (a1a) is such that the amount of water in the liquid mixture is in the range from 10 to 60 wt% based on the total amount of the liquid mixture.

3. The process according to claim 1 or 2, wherein the pressure is reduced in (i) until the suspension has cooled down to a temperature in the range from 10 to 30°C

4. The process according to any of claims 1 to 3, wherein the pressure is reduced in (i) stepwise or continuously.

5. The process according to any of claims 1 to 4, wherein an additional cooling is started after the temperature of the liquid mixture is reduced to a temperature in the range from 50 to 20°C in (a1b) and wherein the crystallization vessel preferably is provided with coolable surfaces for the additional cooling.

6. The process according to claim 1 or 5, wherein the coolable surfaces comprise a cooling jacket, cooling coils, half-pipe coils or cooled baffles.

7. The process according to any of claims 1 to 6, wherein after completing cooling in (a1b) the process is finished and the pressure is set to ambient pressure.

8. The process according to claim 1 or 6, wherein the organic mixture is mixed with water such that the obtained mixture contains 10 to 60 wt% water before bringing the organic mixture into contact with the at least one coolable surface in (a2).

9. The process according to claim 8, wherein the water has a temperature in the range from 40 to 100°C.

10. The process according to any of claims 1 to 9, wherein the mother liquor comprising the organic solvent is separated into an aqueous phase and an organic phase comprising the organic solvent.

11. The process according to any of claims 1 to 10 wherein the organic mixture is obtained by an oxidization reaction of 4,4'-dichlorodiphenyl sulfoxide and an oxidization agent, preferably aqueous hydrogen peroxide with a concentration of 70 to 85 wt%, which is carried out in the organic solvent.

12. The process according to claims 10 and 11, wherein the organic phase is recycled into the oxidization reaction.

13. The process according to any of claims 1 to 12, wherein the linear C₆ to C₁₀ carboxylic acid is n-hexanoic acid, n-heptanoic acid, or a mixture thereof.

14. The process according to any of claims 1 to 13, wherein for initializing crystallization of the 4,4'-dichlorodiphenyl sulfone following steps are carried out before reducing the pressure in step (i):
- reducing the pressure in the crystallization vessel such that the boiling point of the water in the liquid mixture is in the range between 80 and 100°C;
- evaporating water until an initial formation of solids takes place;
- increasing the pressure in the vessel and heating the liquid mixture in the crystallization vessel to a temperature in the range from 1 to 10°C below the saturation point of DCDPS.

15. Use of a gastight closed vessel as crystallization vessel in a process for obtaining 4,4'-dichlorodiphenyl sulfone from an organic mixture comprising 4,4'-dichlorodiphenyl sulfone and an organic solvent which comprises cooling the organic mixture comprising 4,4'-dichlorodiphenyl sulfone and a linear C₆ to C₁₀ carboxylic acid as organic solvent by
- mixing the organic mixture with water in the gastight closed vessel to obtain a liquid mixture;
- cooling the liquid mixture to a temperature below the saturation point of 4,4'-dichlorodiphenyl sulfone by
(i) reducing the pressure in the crystallization vessel to a pressure at which the water starts to evaporate,
(ii) condensing the evaporated water by cooling,
(iii) mixing the condensed water into the liquid mixture in the crystallization vessel, to obtain a suspension comprising crystallized 4,4'-dichlorodiphenyl sulfone.

## Patentansprüche

1. Verfahren zum Gewinnen von 4,4'-Dichlordiphenylsulfon aus einem organischen Gemisch, das 4,4'-Dichlordiphenylsulfon und eine lineare C₆- bis C₁₀-Carbonsäure als organisches Lösungsmittel umfasst, umfassend:
(a) Kühlen des organischen Gemischs durch
(a1a) Mischen des organischen Gemischs mit Wasser in einem Kristallisationsgefäß, um ein flüssiges Gemisch zu erhalten;
(alb) Kühlen des bei (a1a) erhaltenen flüssigen Gemischs auf eine Temperatur unter dem Sättigungspunkt von 4,4'-Dichlordiphenylsulfon durch
(i) Verringern des Drucks in dem Kristallisationsgefäß auf einen Druck, bei dem das Wasser zu verdampfen beginnt,
(ii) Kondensieren des verdampften Wassers durch Kühlen,
(iii) Mischen des kondensierten Wassers in das flüssige Gemisch in dem Kristallisationsgefäß,
um eine Suspension zu erhalten, die kristallisiertes 4,4'-Dichlordiphenylsulfon umfasst;
oder durch
(a2) Inkontaktbringen des organischen Gemischs mit wenigstens einer kühlbaren Oberfläche und dadurch Verringern der Temperatur in dem organischen Gemisch mit einer Kühlrate in dem Bereich von 5 bis 50 K/h, bis eine Temperatur in dem Bereich von 0 bis 30 °C erreicht ist, wobei das organische Gemisch und die wenigstens eine kühlbare Oberfläche eine Temperaturdifferenz aufweisen, die während des gesamten Kühlverfahrens in dem Bereich von 1 bis 30 K gehalten wird, um eine Suspension zu erhalten, die kristallisiertes 4,4'-Dichlordiphenylsulfon umfasst,
(b) Durchführen einer fest-flüssig-Trennung der bei (alb) oder (a2) erhaltenen Suspension, um ein zurückbleibendes feuchtehaltiges festes 4,4'-Dichlordiphenylsulfon als Produkt und Mutterlauge, die das organische Lösungsmittel und Wasser umfasst, zu erhalten.

2. Verfahren gemäß Anspruch 1, wobei die Menge an Wasser, die bei (a1a) zu dem organischen Gemisch gemischt wird, so ist, dass die Menge an Wasser in dem flüssigen Gemisch in dem Bereich von 10 bis 60 Gew.-%, bezogen auf die Gesamtmenge des flüssigen Gemischs, liegt.

3. Verfahren gemäß Anspruch 1 oder 2, wobei der Druck bei (i) verringert wird, bis die Suspension auf eine Temperatur in dem Bereich von 10 bis 30 °C abgekühlt ist.

4. Verfahren gemäß einem der Ansprüche 1 bis 3, wobei der Druck bei (i) schrittweise oder kontinuierlich verringert wird.

5. Verfahren gemäß einem der Ansprüche 1 bis 4, wobei eine zusätzliche Kühlung gestartet wird, nachdem die Temperatur des flüssigen Gemischs bei (alb) auf eine Temperatur in dem Bereich von 50 bis 20 °C verringert worden ist, und wobei das Kristallisationsgefäß vorzugsweise mit kühlbaren Oberflächen für die zusätzliche Kühlung ausgestattet ist.

6. Verfahren gemäß Anspruch 1 oder 5, wobei die kühlbaren Oberflächen einen Kühlmantel, Kühlschlangen, Halbrohrschlangen oder gekühlte Leitbleche umfassen.

7. Verfahren gemäß einem der Ansprüche 1 bis 6, wobei das Verfahren nach Abschließen des Kühlens bei (alb) abgeschlossen ist und der Druck auf Umgebungsdruck eingestellt wird.

8. Verfahren gemäß Anspruch 1 oder 6, wobei das organische Gemisch mit Wasser gemischt wird, so dass das erhaltene Gemisch 10 bis 60 Gew.-% Wasser enthält, bevor das organische Gemisch bei (a2) mit der wenigstens einen kühlbaren Oberfläche in Kontakt gebracht wird.

9. Verfahren gemäß Anspruch 8, wobei das Wasser eine Temperatur in dem Bereich von 40 bis 100 °C aufweist.

10. Verfahren gemäß einem der Ansprüche 1 bis 9, wobei die Mutterlauge, die das organische Lösungsmittel umfasst, in eine wässrige Phase und eine organische Phase, die das organische Lösungsmittel umfasst, aufgetrennt wird.

11. Verfahren gemäß einem der Ansprüche 1 bis 10, wobei das organische Gemisch durch eine Oxidationsreaktion von 4,4'-Dichlordiphenylsulfoxid und einem Oxidationsmittel, vorzugsweise wässrigem Wasserstoffperoxid mit einer Konzentration von 70 bis 85 Gew.-%, die in dem organischen Lösungsmittel durchgeführt wird, erhalten wird.

12. Verfahren gemäß Ansprüchen 10 und 11, wobei die organische Phase in die Oxidationsreaktion recycelt wird.

13. Verfahren gemäß einem der Ansprüche 1 bis 12, wobei die lineare C₆- bis C₁₀-Carbonsäure n-Hexansäure, n-Heptansäure oder ein Gemisch davon ist.

14. Verfahren gemäß einem der Ansprüche 1 bis 13, wobei zum Auslösen der Kristallisation des 4,4'-Dichlordiphenylsulfons die folgenden Schritte vor dem Verringern des Drucks bei Schritt (i) durchgeführt werden:
- Verringern des Drucks in dem Kristallisationsgefäß, so dass der Siedepunkt des Wassers in dem flüssigen Gemisch in dem Bereich zwischen 80 und 100 °C liegt;
- Verdampfen von Wasser, bis erste Entstehung von Feststoffen erfolgt;
- Erhöhen des Drucks in dem Gefäß und Erhitzen des flüssigen Gemischs in dem Kristallisationsgefäß auf eine Temperatur in dem Bereich von 1 bis 10 °C unter dem Sättigungspunkt von DCDPS.

15. Verwendung eines gasdicht verschlossenen Gefäßes als Kristallisationsgefäß bei einem Verfahren zum Gewinnen von 4,4'-Dichlordiphenylsulfon aus einem organischen Gemisch, das 4,4'-Dichlordiphenylsulfon und ein organisches Lösungsmittel umfasst, umfassend Kühlen des organischen Gemischs, das 4,4'-Dichlordiphenylsulfon und eine lineare C₆- bis C₁₀-Carbonsäure als organisches Lösungsmittel umfasst, durch
- Mischen des organischen Gemischs mit Wasser in dem gasdicht verschlossenen Gefäß, um ein flüssiges Gemisch zu erhalten;
- Kühlen des flüssigen Gemischs auf eine Temperatur unter dem Sättigungspunkt von 4,4'-Dichlordiphenylsulfon durch
(i) Verringern des Drucks in dem Kristallisationsgefäß auf einen Druck, bei dem das Wasser zu verdampfen beginnt,
(ii) Kondensieren des verdampften Wassers durch Kühlen,
(iii) Mischen des kondensierten Wassers in das flüssige Gemisch in dem Kristallisationsgefäß, um eine Suspension zu erhalten, die kristallisiertes 4,4'-Dichlordiphenylsulfon umfasst.

## Revendications

1. Procédé d'obtention de 4,4'-dichlorodiphénylsulfone à partir d'un mélange organique comprenant de la 4,4'-dichlorodiphénylsulfone et un acide carboxylique en C₆ à C₁₀ linéaire comme solvant organique, comprenant :
(a) le refroidissement du mélange organique par
(a1a) mélange du mélange organique avec de l'eau dans un récipient de cristallisation pour obtenir un mélange liquide ;
(alb) refroidissement du mélange liquide obtenu dans
(a1a) jusqu'à une température inférieure au point de saturation de la 4,4'-dichlorodiphénylsulfone par
(i) réduction de la pression dans le récipient de cristallisation jusqu'à une pression à laquelle l'eau commence à s'évaporer,
(ii) condensation de l'eau évaporée par refroidissement,
(iii) mélange de l'eau condensée dans le mélange liquide dans le récipient de cristallisation,
pour obtenir une suspension comprenant de la 4,4'-dichlorodiphénylsulfone cristallisée ;
ou par
(a2) le fait d'amener le mélange organique en contact avec au moins une surface refroidissable et ainsi la réduction de la température dans le mélange organique avec une vitesse de refroidissement dans la plage de 5 à 50 K/h jusqu'à ce qu'une température dans la plage de 0 à 30 °C soit atteinte, dans lequel le mélange organique et l'au moins une surface refroidissable ont une différence de température qui est maintenue pendant tout le procédé de refroidissement dans la plage de 1 à 30 K pour obtenir une suspension comprenant de la 4,4'-dichlorodiphénylsulfone cristallisée ;
(b) la réalisation d'une séparation solide-liquide de la suspension obtenue en (alb) ou en (a2) pour obtenir une 4,4'-dichlorodiphénylsulfone solide contenant de l'humidité résiduelle comme produit et de la liqueur mère comprenant le solvant organique et de l'eau.

2. Procédé selon la revendication 1, dans lequel la quantité d'eau mélangée au mélange organique en (a1a) est telle que la quantité d'eau dans le mélange liquide est dans la plage de 10 à 60 % en poids, sur la base de la quantité totale du mélange liquide.

3. Procédé selon la revendication 1 ou 2, dans lequel la pression est réduite en (i) jusqu'à ce que la suspension soit refroidie jusqu'à une température dans la plage de 10 à 30 °C.

4. Procédé selon l'une quelconque des revendications 1 à 3, dans lequel la pression est réduite en (i) par étapes ou de manière continue.

5. Procédé selon l'une quelconque des revendications 1 à 4, dans lequel un refroidissement supplémentaire est démarré après que la température du mélange liquide est réduite jusqu'à une température dans la plage de 50 à 20 °C dans (alb) et dans lequel le récipient de cristallisation est de préférence pourvu de surfaces refroidissables pour le refroidissement supplémentaire.

6. Procédé selon la revendication 1 ou 5, dans lequel les surfaces refroidissables comprennent une enveloppe de refroidissement, des bobines de refroidissement, des bobines de demi-tuyau ou des déflecteurs refroidis.

7. Procédé selon l'une quelconque des revendications 1 à 6, dans lequel après avoir terminé le refroidissement en (alb) le procédé est terminé et la pression est réglée à la pression ambiante.

8. Procédé selon la revendication 1 ou 6, dans lequel le mélange organique est mélangé avec de l'eau de telle sorte que le mélange obtenu contient 10 à 60 % en poids d'eau avant d'amener le mélange organique en contact avec l'au moins une surface refroidissable en (a2).

9. Procédé selon la revendication 8, dans lequel l'eau a une température dans la plage de 40 à 100 °C.

10. Procédé selon l'une quelconque des revendications 1 à 9, dans lequel la liqueur mère comprenant le solvant organique est séparée en une phase aqueuse et une phase organique comprenant le solvant organique.

11. Procédé selon l'une quelconque des revendications 1 à 10 dans lequel le mélange organique est obtenu par une réaction d'oxydation de sulfoxyde de 4,4'-dichlorodiphényle et d'un agent d'oxydation, de préférence du peroxyde d'hydrogène aqueux avec une concentration de 70 à 85 % en poids, qui est effectuée dans le solvant organique.

12. Procédé selon les revendications 10 et 11, dans lequel la phase organique est recyclée dans la réaction d'oxydation.

13. Procédé selon l'une quelconque des revendications 1 à 12, dans lequel l'acide carboxylique en C₆ à C₁₀ linéaire est l'acide n-hexanoïque, l'acide n-heptanoïque ou un mélange correspondant.

14. Procédé selon l'une quelconque des revendications 1 à 13, dans lequel pour initialiser la cristallisation de la 4,4'-dichlorodiphénylsulfone les étapes suivantes sont effectuées avant de réduire la pression à l'étape (i) :
- réduction de la pression dans le récipient de cristallisation de sorte que le point d'ébullition de l'eau dans le mélange liquide soit dans la plage comprise entre 80 et 100 °C ;
- évaporation de l'eau jusqu'à ce qu'une formation initiale de solides ait lieu ;
- augmentation de la pression dans le récipient et chauffage du mélange liquide dans le récipient de cristallisation jusqu'à une température dans la plage de 1 à 10 °C en dessous du point de saturation du DCDPS.

15. Utilisation d'un récipient fermé étanche aux gaz comme récipient de cristallisation dans un procédé d'obtention de 4,4'-dichlorodiphénylsulfone à partir d'un mélange organique comprenant de la 4,4'-dichlorodiphénylsulfone et un solvant organique qui comprend le refroidissement du mélange organique comprenant de la 4,4'-dichlorodiphénylsulfone et un acide carboxylique en C₆ à C₁₀ linéaire comme solvant organique par
- mélange du mélange organique avec de l'eau dans le récipient fermé étanche aux gaz pour obtenir un mélange liquide ;
- refroidissement du mélange liquide jusqu'à une température inférieure au point de saturation de la 4,4'-dichlorodiphénylsulfone par
(i) réduction de la pression dans le récipient de cristallisation jusqu'à une pression à laquelle l'eau commence à s'évaporer,
(ii) condensation de l'eau évaporée par refroidissement,
(iii) mélange de l'eau condensée dans le mélange liquide dans le récipient de cristallisation, afin d'obtenir une suspension comprenant de la 4,4'-dichlorodiphénylsulfone cristallisée.
